# EUROPÄISCHE PATENTSCHRIFT

(11) **EP 2 289 539 B1**
(45) Veröffentlichungstag und Bekanntmachung des Hinweises auf die Patenterteilung: **19.09.2012**
(21) Anmeldenummer: 10178836.2
(22) Anmeldetag: 09.03.2002
(51) Int. Cl.: A61K 38/28, A61K 47/00

(54) **Zinkfreie und zinkarme Insulinzubereitungen mit verbesserter Stabilität**
Zinc-free and low-zinc insulin preparations with improved stability
Préparations d'insuline sans zinc ou pauvre en zinc dotées d'une stabilité améliorée

(30) Priorität: 23.03.2001 DE 10114178
(43) Veröffentlichungstag der Anmeldung: 02.03.2011
(62) Teilanmeldung aus: 02729985.8
(73) Patentinhaber: Sanofi-Aventis Deutschland GmbH, 65929 Frankfurt am Main (DE)
(72) Erfinder: Boderke, Peter, 65824 Schwalbach (DE)

(56) Entgegenhaltungen:
- EP-A- 0 166 529
- EP-A- 0 200 383
- WO-A-00/74736
- WO-A-98/42749
- US-A- 4 153 689
- US-A- 5 506 203
- LOUGHEED W D ET AL: "Physical stability of insulin formulations.", DIABETES. UNITED STATES MAY 1983, Bd. 32, Nr. 5, Mai 1983 (1983-05), Seiten 424-432, XP001096187, ISSN: 0012-1797
- THUROW H ET AL: "Stabilisation of dissolved proteins against denaturation at hydrophobic interfaces.", DIABETOLOGIA. GERMANY, WEST AUG 1984, Bd. 27, Nr. 2, August 1984 (1984-08), Seiten 212-218, XP001096189, ISSN: 0012-186X

## Beschreibung

Die Erfindung betrifft stabilisierte pharmazeutische Formulierungen enthaltend Asp (B28) Humaninsulin oder Lys(B28) Pro(B29) Humaninsulin; ein Tensid oder Kombinationen mehrerer Tenside, wobei das Tensid Partial- und Fettsäureester und-ether mehrwertiger Alkohole, des Glycerols und des Sorbitols darstellt; und optional ein Konservierungsmittel oder Kombinationen mehrerer Konservierungsmittel sowie optional ein Isotonisierungsmittel, Puffer oder weitere Hilfsstoffe oder Kombinationen davon, wobei die pharmazeutische Formulierung zinkarm oder frei von Zink ist. Diese Formulierungen können zur Behandlung von Diabetes eingesetzt werden und sind besonders für den Einsatz in Insulinpumpen, Pens, Injektoren, Inhalatoren oder für Zubereitungen, bei denen eine erhöhte physikalische Stabilität erforderlich ist, einsetzbar. Die Erfindung betrifft ebenfalls parenterale Zubereitungen, die solche Formulierungen enthalten und bei Diabetes angewendet werden können sowie Methoden, um die Zubereitungen herzustellen und die Stabilität von Insulinzubereitungen zu verbessern.

Weltweit leiden etwa 120 Mio. Menschen an Diabetes mellitus. Darunter sind etwa 12 Mio. Typ I-Diabetiker, für die die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen.

Beim Gesunden ist die Insulinfreisetzung durch den Pankreas strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subcutane Applikation von Insulin erreicht in der Regel die oben beschriebene Qualität der physiologischen Regulation der Blutglucose nicht annähernd. Häufig kommt es zu Entgleisungen der Blutglucose nach oben oder unten, die in ihren schwersten Formen lebensbedrohlich sein können. Daneben stellen jedoch auch über Jahre erhöhte Blutglucosespiegel ohne anfängliche Symptome ein erhebliches Gesundheitsrisiko dar. Die großangelegte DCCT-Studie in den USA (The Diabetes Control and Complications Trial Research Group (1993) N. Engl. J. Med. 329, 977-986) wies eindeutig nach, daß chronisch erhöhte Blutglucosespiegel wesentlich für die Entwicklung diabetischer Spätschäden verantwortlich sind. Diabetische Spätschäden sind mikro- und makrovaskuläre Schädigungen, die sich u.U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, daß eine verbesserte Therapie des Diabetes in erster Linie darauf abzielen muß, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäureresten und/oder Addition/Entfernen wenigstens eines Aminosäurerestes von dem entsprechenden, ansonsten gleichen natürlich vorkommenden Insulin unterscheiden. Es kann sich bei den hinzugefügten und / oder ersetzten Aminosäreresten auch um solche handeln, die nicht natürlich vorkommen.

Insulinderivate sind Derivate von natürlich vorkommendem Insulin oder einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen.

In der Regel haben Insulinderivate und Insulinanaloga gegenüber humanem Insulin eine etwas veränderte Wirkung.

Insulinanaloga mit beschleunigtem Wirkungseintritt werden in EP 0 214 826, EP 0 375 437 und EP 0 678 522 beschrieben. EP 0 124 826 bezieht sich u.a. auf Substitutionen von B27 und B28. EP 0 678 522 beschreibt Insulinanaloga, die in der Position B29 verschiedene Aminosäuren, vorzugsweise Prolin, aufweisen, jedoch nicht Glutaminsäure.

EP 0 375 437 umfaßt Insulinanaloga mit Lysin oder Arginin in B28, die optional zusätzlich in B3 und/oder A21 modifiziert sein können.

In der EP 0 419 504 werden Insulinanaloga offenbart, die gegen chemische Modifikationen geschützt sind, in dem Asparagin in B3 und wenigstens eine weitere Aminosäure in den Positionen A5, A15, A18 oder A21 verändert sind.

In der WO 92/00321 werden Insulinanaloga beschrieben, bei denen wenigstens eine Aminosäure der Positionen B1-B6 durch Lysin oder Arginin ersetzt ist. Derartige Insuline weisen gemäß WO 92/00321 eine verlängerte Wirkung auf.

Die auf dem Markt befindlichen Insulinzubereitungen von natürlich vorkommenden Insulinen zur Insulinsubstitution unterscheiden sich in der Herkunft des Insulins (z.B. Rind, Schwein, Humaninsulin), sowie der Zusammensetzung, womit das Wirkprofil (Wirkeintritt und Wirkdauer) beeinflusst werden kann. Durch Kombination verschiedener Insulinpräparate lassen sich unterschiedlichste Wirkprofile erzielen und möglichst physiologische Blutzuckerwerte einstellen. Seit einiger Zeit sind nicht nur die genannten natürlich vorkommenden Insuline, sondern auch Zubereitungen von Insulinderivaten oder Insulinanaloga auf dem Markt, die eine veränderte Kinetik zeigen. Die rekombinante DNA Technologie ermöglicht heutzutage die Herstellung von solchen modifizierten Insulinen. Hierzu zählen sogenannte monomere Insulinanaloga wie Insulin Lispro, Insulin Aspart und HMR 1964 (Lys(B3), Glu(B29) Humaninsulin) mit einem schnellen Wirkeintritt, sowie Insulin Glargin mit einer verlängerten Wirkdauer.

Neben der Wirkdauer ist die Stabilität der Zubereitung für die Patienten sehr wichtig. Stabilisierte Insulinformulierungen mit erhöhter physikalischer Langzeitstabilität werden insbesondere für Zubereitungen benötigt, die besonderen mechanischen Belastungen oder höheren Temperaturen ausgesetzt sind. Hierzu zählen z.B. Insuline in Applikationssystemen wie Pens, Inhalationssystemen, nadellosen Injektionssystemen oder Insulinpumpen. Insulinpumpen werden entweder am Körper des Patienten getragen oder implantiert. In beiden Fällen ist die Zubereitung der Körperwärme und Bewegung sowie der Förderbewegung der Pumpe und damit einer sehr hohen thermomechanischen Belastung ausgesetzt. Da auch Insulinpens (Einmal- oder wiederverwertbare Pens) meist am Körper getragen werden, gilt hier das gleiche. Bisherige Zubereitungen haben nur eine limitierte Stabilität unter diesen Bedingungen. Insulin liegt in neutraler Lösung in pharmazeutischer Konzentration in Form stabilisierter zinkhaltiger Hexamere vor, die aus 3 identischen Dimereinheiten aufgebaut sind (Brange et al., Diabetes Care 13:923-954 (1990)). Durch Veränderung der Aminosäuresequenz kann die Assoziation von Insulin verringert werden. Somit liegt z.B. das Insulinanalogon Lispro vorwiegend als Monomer vor und wird dadurch schneller resorbiert und zeigt eine kürzere Wirkdauer (HPT Ammon und C. Werning; Antidiabetika; 2. Aufl.; Wiss. Verl.-Ges. Stuttgart; 2000; S. 94.f). Gerade die schnell wirksamen Insulinanaloga, die in Monomeren- oder Dimerenform vorliegen, zeigen jedoch eine verringerte Stabilität und erhöhte Aggregationsneigung bei thermischer und mechanischer Belastung. Sie macht sich häufig in Trübungen und Ausfällungen von unlöslichen Aggregaten bemerkbar. (Bakaysa et al, US Patent Nr. 5474978). Diese höhermolekularen Transformationsprodukte (Dimere, Trimere, Polymere) und Aggregate verringern nicht nur die applizierte Insulindosis sondern können auch Irritationen oder Immunreaktionen beim Patienten auslösen. Ausserdem können solche unlöslichen Aggregate die Kanülen und Schläuche der Pumpen zusetzen und verstopfen. Da Zink zu einer zusätzlichen Stabilisierung des Insulins führt, sind zinkfreie oder zinkarme Zubereitungen von Insulin und Insulinanaloga besonders anfällig für Instabilitäten. Insbesondere monomere Insulinanaloga mit einem schnellen Wirkeintritt neigen sehr schnell zur Aggregation und physikalischen Instabilitäten, weil die Bildung von unlöslichen Aggregaten über Monomere des Insulins abläuft. Um die Qualität einer Insulinzubereitung zu gewährleisten, ist es notwendig, die Bildung von Aggregaten zu vermeiden.
Es gibt verschiedene Ansätze, Insulinformulierungen zu stabilisieren. So sind in der internationalen Patentanmeldung WO98/56406 stabilisierte Formulierungen durch TRIS oder Arginin Puffer beschrieben worden. Patent US 5866538 beschreibt eine Insulin-Zubereitung, die Glycerol und Natriumchlorid in Konzentrationen von 5 - 100 mM enthält und eine erhöhte Stabilität aufweisen soll. US Patent 5948751 beschreibt Insulinzubereitungen mit erhöhter physikalischer Stabilität, die durch Zusatz von Mannitol oder ähnlichen Zuckern erreicht wird. Der Zusatz von überschüssigem Zink zu einer zinkhaltigen Insulinlösung kann ebenfalls die Stabilität erhöhen (J. Brange et al., Diabetic Medicine, 3: 532-536, 1986). Auch der Einfluss des pH Wertes und verschiedener Hilfsstoffe auf die Stabilität von Insulinzubereitungen wurde ausführlich beschrieben (J. Brange & L. Langkjaer, Acta Pharm. Nordica 4: 149-158).
Oft reichen diese Stabilisierungsmethoden für erhöhte Anforderungen (Verbesserung der Haltbarkeit bei Raum- oder Körpertemperatur und mechanischer Belastung) oder für sogenannte monomere Insulinanaloga bzw. schnell wirksame Insuline, die besonders anfällig für physikalischen Stress sind, nicht aus. Zudem enthalten alle kommerziellen Insulinzubereitungen Zink, welches zugesetzt wird, um die Zubereitung zu stabilisieren. So beschreibt Bakaysa et al. im US Patent 5474978 stabilisierte Formulierungen aus Insulinkomplexen, die aus 6 Insulinanalogmonomeren, 2 Zinkatomen und mindestens 3 Molekülen eines phenolischen Konservierungsmittels bestehen. Zusätzlich können diese Formulierungen einen physiologisch akzeptablen Puffer und ein Konservierungsmittel enthalten. Will man hingegen zinkfreie oder zinkarme Insulinzubereitungen herstellen, so sind die genannten Stabilisierungsmethoden nicht ausreichend für eine vermarktungsfähige Zubereitung. Beispielsweise konnte eine zinkfreie Zubereitung von Insulin Lispro aufgrund unzureichender physikalischer Stabilität nicht entwickelt werden (Bakaysa et al., Protein Science (1996), 5:2521-2531). Zinkarme oder zinkfreie Insulinformulierungen mit ausreichender Stabilität, insbesondere physikalischer Stabilität sind im Stand der Technik nicht beschrieben.

Der vorliegenden Erfindung lag somit die Aufgabe zugrunde, zinkfreie Zubereitungen für Insuline und deren Derivate und Analoga zu finden, welche sich durch eine hohe Stabilität auszeichnen.

Es wurde nun überraschenderweise gefunden, dass der Zusatz von Tensiden (Emulgatoren) wie z.B. Poloxamere oder Polysorbate (Tween^{®}) die Stabilität von Insulinzubereitungen drastisch erhöhen kann und somit sogar zinkfreie Zubereitungen hergestellt werden können, die eine überlegene Stabilität aufweisen, um auch in Infusionspumpen oder anderen Applikationssystemen verwendet werden zu können. Besonders unter Stressbedingungen zeigen diese Zubereitungen erhöhte Stabilität. Dies gilt sowohl für Insulin, Insulinanaloga, Insulinderivate oder Mischungen daraus.

Insulin bildet in neutralen Zubereitungen mit Zinkionen Komplexe. Hierbei bilden sich bei ausreichender Zinkkonzentration aus 6 Insulinmolekülen und 2 Zinkionen stabile Hexamere. Zur Ausbildung dieser Struktur ist eine Zinkkonzentration von mindestens 0,4 % (w/w) bezogen auf das Insulin erforderlich. Dies entspricht bei einer Zubereitung von 100 IU/ml Insulin einer Konzentration von ca. 13 µg/ml Zink. Ein Überschuss an Zink (z.B. 4 Zinkionen pro Hexamer) stabilisiert die Zubereitung gegenüber physikalischem Stress nochmals deutlich (J. Brange et al., Neutral insulin solutions physically stabilized by the addition of Zn2+. Diabetic Med. 3, 532-536 (1986)). Im Gegensatz dazu ist in Zubereitungen mit geringeren Zinkkonzentrationen (< 0,4 Gewichtsprozent bezogen auf Insulin) die Bildung der Hexamere reduziert. Dies führt zu einer dramatisch reduzierten Stabilität der Zubereitung (J. Brange and L. Langkjaer; Acta Pharm Nord, 4: 149-158 (1992)). "Zinkfrei" oder "Zinkarm" im Sinne dieser Anmeldung bedeutet daher das Vorhandensein von weniger als 0,4 Gewichtsprozent Zink bezogen auf den Insulingehalt der Zubereitung, vorzugsweise weniger als 0,2 Gewichtsprozent bezogen auf den Insulingehalt. Für eine gängige Insulinzubereitung mit 100 Einheiten pro Milliliter (0,6 µmol/ml) bedeutet dies zum Beispiel eine Konzentration von weniger als 13 µg/ml Zn⁺⁺-Ionen (0,2 µmol/ml), vorzugsweise weniger als 6,5 µg/ml Zn⁺⁺-Ionen in der pharmazeutischen Zubereitung, bezogen auf eine Insulinkonzentration von 100 Einheiten/ml. Die Zinkfreiheit kann auch durch Zugabe von Zink komplexierenden Stoffen, wie z.B. Citrat oder EDTA erreicht werden, sodass nicht ausreichend Zinkionen zur Bildung des Insulin/Zink Hexamerenkomplexes zur Verfügung stehen.

Die pharmazeutischen Zubereitungen enthalten 60-6000 nmol/ml, bevorzugt 240-3000 nmol/ml eines Insulins gemäß Anspruch 1.

Als Tenside werden Tenside gemäß Anspruch 1 verwendet, wie z.B.:
Partial- und Fettsäureester mehrwertiger Alkohole wie des Glycerols, Sorbitols u.ä. (Span^{®}, Tween^{®}, nMyrj^{®}, Brij^{®}).
Die Tenside liegen in der pharmazeutischen Zusammensetzung in einer Konzentration von 0,1 µg/ml - 10000 µg/ml, bevorzugt 1 µg/ml - 1000 µg/ml vor.

Die Zubereitung kann desweiteren Konservierungsmittel (z.B. Phenol, Kresol, Parabene), Isotonisierungsmittel (z.B. Mannitol, Sorbitol, Lactose, Dextrose, Trehalose, Natriumchlorid, Glycerol) Puffersubstanzen, Salze, Säuren und Laugen sowie weitere Hilfstoffe enthalten. Diese Substanzen können jeweils einzeln oder auch als Mischungen vorliegen.

Glycerol, Dextrose, Lactose, Sorbitol und Mannitol liegen üblicherweise in der pharmazeutischen Zubereitung in einer Konzentration von 100 - 250 mM, NaCl in einer Konzentration bis zu 150 mM vor. Puffersubstanzen, wie z.B. Phosphat-, Acetat-, Citrat, Arginin , Glycylglycin oder TRIS (d.h. 2-Amino-2-Hydroxymethyl-1,3-Propandiol) Puffer sowie entsprechende Salze, sind in einer Konzentration von 5 - 250 mM, bevorzugt 10 - 100 mM vorhanden.
Weitere Hilfstoffe können unter anderem sein Salze, Arginin, Protamin, oder Surfen^{®}.

Gegenstand der Erfindung ist daher eine pharmazeutische Formulierung gemäß Anspruch 1 enthaltend optional ein Konservierungsmittel oder Kombinationen mehrerer Konservierungsmittel; und optional ein Isotonisierungsmittel, Puffersubstanzen und/oder weitere Hilfsstoffe oder Kombinationen davon, wobei die pharmazeutische Formulierung frei von oder arm an Zink ist; bevorzugt ist eine solche pharmazeutische Formulierung, wobei die Partial- und Fettsäureester und -ether des Glycerols und Sorbitols ausgewählt werden aus einer Gruppe enthaltend Span^{®}, Tween^{®}, Myrj^{®}, Brij^{®}, Cremophor^{®}; wobei das Konservierungsmittel ausgewählt wird aus einer Gruppe enthaltend Phenol, Kresol, Parabene; wobei das Isotonisierungsmittel ausgewählt wird aus einer Gruppe enthaltend Mannitol, Sorbitol, Natriumchlorid, Glycerol; wobei die Hilfsstoffe ausgewählt werden aus einer Gruppe enthaltend Puffersubstanzen, Säuren, Laugen; wobei das Insulin Lys^{B28}Pro^{B29} Humaninsulin oder B28 Asp-Humaninsulin ist.

Ein weiterer Gegenstand der Erfindung ist eine pharmazeutische Formulierung wie oben beschrieben, bei der das Insulinanalogon in einer Konzentration von 60 - 6000 nmol/ml, vorzugsweise in einer Konzentration von 240 - 3000 nmol/ml vorliegt (dies entspricht etwa einer Konzentration von 1,4 - 35 mg/ml oder 40 - 500 Einheiten/ml); bei der das Tensid in einer Konzentration von 0,1 - 10000 µg/ml, vorzugsweise in einer Konzentration von 1 - 1000 µg/ml vorliegt.

Ein weiterer Gegenstand der Erfindung ist eine pharmazeutische Formulierung wie oben ausgeführt, bei der Glycerol und/oder Mannitol in einer Konzentration von 100 - 250 mM, und/oder Chlorid vorzugsweise in einer Konzentration bis zu 150 mM vorliegt.

Ein weiterer Gegenstand der Erfindung ist eine pharmazeutische Formulierung wie oben ausgeführt, bei der eine Puffersubstanz in einer Konzentration von 5 - 250 mM vorliegt.
Ein weiterer Gegenstand der Erfindung ist eine pharmazeutische Insulinformulierung, die weitere Zusätze wie z.B. Salze, Protamin oder Surfen^{®} enthält, welche die Freigabe von Insulin retardieren. Auch Mischungen aus solchen Retardinsulinen mit oben beschriebenen Formulierungen sind darin eingeschlossen.
Ein weiterer Gegenstand der Erfindung ist eine Methode zur Herstellung solcher pharmazeutischer Formulierungen. Ebenso erlaubt die Erfindung die Anwendung solcher Formulierungen zur Behandlung von Diabetes mellitus.
Ein weiterer Gegenstand der Erfindung ist die Verwendung bzw. der Zusatz der beanspruchten Tenside als Stabilisator während des Herstellungsprozesses des Insulinanalogons.

Bei den beschriebenen pharmazeutischen Formulierungen enthaltend ein Polypeptid gemäß Anspruch 1 liegt der pH-Wert zwischen 2 und 12, bevorzugt zwischen 6 und 8,5 und besonders bevorzugt zwischen 7 und 7,8.

Im folgenden wird die Anmeldung anhand einiger (Vergleichs-) Beispiele beschrieben, die keinesfalls beschränkend wirken sollen.

### Beispiele:

Vergleichsuntersuchungen: Es werden verschiedene zinkfreie Zubereitungen mit dem Insulinanalogon HMR1964 (Lys(B3), Glu(B29), Humaninsulin) hergestellt. Dazu wird zinkfreies HMR1964 und die übrigen Bestandteile in einem Teil Wasser für Injektionszwecke gelöst und der pH Wert mit Salzsäure/NaOH auf 7,3 +/- 0,2 eingestellt und auf das Endvolumen aufgefüllt. Die Konzentration von HMR 1964 beträgt bei jedem der unten beschriebenen Versuche 3,5 mg/ml (entspricht 100 Einheiten/ml). Eine zweite Zubereitung wird identisch hergestellt, jedoch wird noch eine bestimmte Menge eines Tensids hinzugegeben. Die Lösungen werden in 5 ml oder 10 ml Glasgefäße (Vials) abgefüllt und verbördelt. Diese Gefässe werden nun Stressbedingungen ausgesetzt:
1. Rotationstest: Jeweils 5 Gefäße einer Charge sowie 5 Gefäße der Vergleichscharge werden einem Rotationstest unterzogen. Hierzu werden die Gefäße in einen Rotator gespannt und bei 37°C bei 60 U/min über Kopf (360°) rotiert. Nach definierten Zeiten wird die Trübung der in den Gefäßen befindlichen Zubereitungen gegen Trübungsstandards verglichen oder mit einem Labortrübungsphotometer (Nephelometer) in Formazin Nephelometrischen Einheiten (FNU) bestimmt. Der Versuch wird solange durchgeführt, bis in allen Gefässen ein Trübungswert von 18 FNU überschritten wird.
2. Schütteltest: Die Gefäße werden auf einen Laborschüttler in einem Inkubator gestellt und bei 30°C mit 100 Bewegungen/min geschüttelt. Nach definierten Zeiten wird der Trübungswert der Proben mittels eines Labor-Trübungsphotometers (Nephelometer) in Formazin Nephelometrischen Einheiten (FNU) bestimmt.

### Beispiel 1: Stabilisierung von HMR1964 durch Zusatz von Zink im Rotationstest

a) In einer wässrigen Lösung, die in der finalen Formulierung 2,7 mg/ml m-Kresol, 20 mg/ml Glycerol und 6 mg/ml Trometamol (Tris) enthält, wird zinkfreies HMR1964 (so berechnet, dass in der fertigen Formulierung eine Konzentration von 3,5 mg/ml entsteht) gelöst und der pH Wert mit 1 N Salzsäure/1 N NaOH auf 7,2 - 7,4 (gemessen bei Raumtemperatur) eingestellt. Die Lösung wird mit Wasser zum Endvolumen aufgefüllt und über einen 0,2 µm Filter steril filtriert. Anschliessend wird sie in 5 ml Injektionsfläschchen gefüllt und mit Kappen verschlossen.
b) Eine Vergleichslösung wird identisch hergestellt, jedoch wird vor dem Auffüllen mit Wasser eine entsprechende Menge einer 0,1 %igen Zinkchlorid Stammlösung hinzugefügt, sodass sich in der fertigen Formulierung ein Zinkgehalt von 15 µg/ml ergibt.

Jeweils 5 Muster werden anschliessend im Rotationstest gestresst und die Trübung nach verschiedenen Zeiträumen bestimmt. Die Ergebnisse sind in nachfolgender Tabelle wiedergegeben.

| | **Anzahl Prüfmuster mit Trübung > 18 FNU nach:** | | | | | |
|---|---|---|---|---|---|---|
| Beschreibung | 0 h | 8 h | 16 h | 32 h | 40 h | 56 h |
| HMR1964 Ohne Zusatz | 0 | 5 | - | - | - | - |
| HMR1964 +15 µg/ml Zn | 0 | 0 | 0 | 0 | 4 | 5 |

Der Zusatz von Zink kann die entstehende Trübung der Lösung zeitlich deutlich verzögern und stabilisiert dadurch die HMR1964 Formulierung. Ohne Zusatz von Zink weist die Zubereitung schon nach 8 Stunden im Rotationstest eine deutliche Trübung auf.

### Beispiel 2: Stabilisierung von HMR1964 durch Zusatz von Polysorbat 20 (Tween® * 20) im Rotationstest

a) Zu einer wässrigen Lösung, die in der finalen Formulierung 3,15 mg/ml m-Kresol, 5 mg/ml NaCl und 6 mg/ml Trometamol enthält wird zinkfreies HMR1964 (so berechnet, dass in der fertigen Formulierung eine Konzentration 3,5 mg/ml entsteht) gelöst und der pH Wert mit 1 N Salzsäure/1 N NaOH auf 7,2 - 7,4 (gemessen bei Raumtemperatur) eingestellt. Die Lösung wird mit Wasser zum Endvolumen aufgefüllt und über einen 0,2 µm Filter steril filtriert. Anschliessend wird sie in 5 ml Injektionsfläschchen gefüllt und mit Kappen verschlossen.
b) Eine Vergleichslösung wird identisch hergestellt, jedoch wird vor dem Auffüllen mit Wasser eine entsprechende Menge einer 0,1 %igen Polysorbat 20 (Tween^{®} 20) Stammlösung hinzugefügt, sodass sich in der fertigen Formulierung eine Konzentration von 10 µg/ml ergibt.

Jeweils 5 Muster werden anschliessend im Rotationstest gestresst und die Trübung nach verschiedenen Zeiträumen bestimmt. Die Ergebnisse sind in nachfolgender Tabelle wiedergegeben.

| | **Anzahl Prüfmuster mit Trübung > 18 FNU nach:** | | | | | |
|---|---|---|---|---|---|---|
| Beschreibung | 0 h | 8 h | 16 h | 24 h | 32 h | 40 h |
| HMR1964 Ohne Zusatz | 0 | 5 | - | - | - | - |
| HMR1964 + 10 µg/ml Tween^{®} 20 | 0 | 0 | 0 | 0 | 5 | - |

Der Zusatz von Polysorbat 20 verzögert das Auftreten von Trübungen sehr deutlich.

### Beispiel 3: Stabilisierung von HMR1964 durch Zusatz von Poloxamer im Rotationstest

a) Zu einer wässrigen Lösung, die in der finalen Formulierung 4,5 mg/ml Phenol, 5 mg/ml NaCl und 6 mg/ml Trometamol enthält wird zinkfreies HMR1964 (so berechnet, dass in der fertigen Formulierung eine Konzentration 3,5 mg/ml entsteht) gelöst und der pH Wert mit 1 N Salzsäure/1 N NaOH auf 7,2 - 7,4 (gemessen bei Raumtemperatur) eingestellt. Die Lösung wird mit Wasser zum Endvolumen aufgefüllt und über einen 0,2 µm Filter steril filtriert. Anschliessend wird sie in 5 ml Injektionsfläschchen gefüllt und mit Kappen verschlossen.
b) Eine Vergleichslösung wird identisch hergestellt, jedoch wird vor dem Auffüllen mit Wasser eine entsprechende Menge einer 0,1 %igen Poloxamer 171 (z.B. Genapol^{®}) Stammlösung hinzugefügt, sodass sich in der fertigen Formulierung eine Konzentration von 10 µg/ml ergibt.

Jeweils 5 Muster werden anschliessend im Rotationstest gestresst und die Trübung nach verschiedenen Zeiträumen bestimmt. Die Ergebnisse sind in nachfolgender Tabelle wiedergegeben.

| | **Anzahl Prüfmuster mit Trübung > 18 FNU nach:** | | | | | |
|---|---|---|---|---|---|---|
| Beschreibung | 0 h | 8 h | 16 h | 24 h | 32 h | 40 h |
| HMR1964 ohne Zusatz | 0 | 5 | - | - | - | - |
| HMR1964 + 0.01 mg/ml Poloxamer 171 | 0 | 0 | 0 | 2 | 5 | - |

Auch der Zusatz von Poloxamer 171 verzögert das Auftreten von Trübungen deutlich und stabilisiert die Zubereitung.

### Beispiel 4: Stabilisierung von HMR1964 durch Zusatz von Polysorbat 20 bzw. Polysorbat 80 im Schütteltest

a) Zu einer wässrigen Lösung, die in der finalen Formulierung 3,15 mg/ml m-Kresol, 5 mg/ml NaCl und 6 mg/ml Trometamol enthält wird zinkfreies HMR1964 (so berechnet, dass in der fertigen Formulierung eine Konzentration von 3,5 mg/ml entsteht) gelöst und der pH Wert mit 1 N Salzsäure/1 N NaOH auf 7,2 - 7,4 (gemessen bei Raumtemperatur) eingestellt. Die Lösung wird mit Wasser zum Endvolumen aufgefüllt und über einen 0,2 µm Filter steril filtriert. Anschliessend wird sie in 5 ml Injektionsfläschchen gefüllt und mit Kappen verschlossen.
b) Eine Vergleichslösung wird identisch hergestellt, jedoch wird vor dem Auffüllen mit Wasser eine entsprechende Menge einer 0,1 %igen Polysorbat 20 (Tween^{®} 20) Stammlösung hinzugefügt, sodass sich in der fertigen Formulierung eine Konzentration von 10 µg/ml ergibt.
b) Eine weitere Vergleichslösung wird identisch wie unter b) hergestellt, diesmal wird jedoch anstelle von Polysorbat 20 Polysorbat 80 (Tween^{®} 80) verwendet.

Die Proben werden bei 30°C auf einem Laborschüttler geschüttelt (60 U/min) und die Trübung der Proben nach bestimmten Zeiten gemessen. Die Ergebnisse sind in nachfolgender Tabelle wiedergegeben.

| | **Schütteltest, Trübung (FNU) nach** | | | | |
|---|---|---|---|---|---|
| **Zusatz** | **Start** | **1 Woche** | **2 Woche** | **3 Woche** | **4 Woche** |
| Ohne Zusatz | 0,55 | 2,04 | 4,86 | 6,12 | 10,51 |
| 0,01 mg/ml Tween 20 | 1,75 | 2,60 | 2,44 | 2,44 | 3,80 |
| 0,01 mg/ml Tween 80 | 2,38 | 2,98 | 2,86 | 3,01 | 4,14 |

Sowohl der Zusatz von Polysorbat 20 als auch von Polysorbat 80 haben einen stabilisierenden Effekt auf HMR1964 im Schütteltest.

### Beispiel 5: Stabilisierung von HMR1964 durch Zusatz von Zink oder Poloxamer (Genapo^{®}) im Schütteltest

a) Zu einer wässrigen Lösung, die in der finalen Formulierung 3,3 mg/ml Phenol, 5 mg/ml NaCl und 6 mg/ml Trometamol enthält wird zinkfreies HMR1964 (so berechnet, dass in der fertigen Formulierung eine Konzentration von 3,5 mg/ml entsteht) gelöst und der pH Wert mit 1 N Salzsäure/1 N NaOH auf 7,2 - 7,4 (gemessen bei Raumtemperatur) eingestellt. Die Lösung wird mit Wasser zum Endvolumen aufgefüllt und über einen 0,2 µm Filter steril filtriert. Anschliessend wird sie in 5 ml Injektionsfläschchen gefüllt und mit Kappen verschlossen.
b) Eine Vergleichslösung wird identisch hergestellt, jedoch wird vor dem Auffüllen mit Wasser eine entsprechende Menge einer 0,1 %igen Poloxamer 171 (Genapol^{®}) Stammlösung hinzugefügt, sodass sich in der fertigen Formulierung ein Gehalt von 10 µg/ml ergibt.
c) Eine weitere Vergleichslösung wird wie unter a) beschrieben hergestellt, jedoch wird anstelle von Poloxamer der Lösung vor dem Auffüllen mit Wasser eine entsprechende Menge einer 0,1 %igen Zinkchlorid Stammlösung hinzugefügt, sodass sich in der fertigen Formulierung eine Konzentration von 15 µg/ml Zink ergibt.

| | **Schütteltest, Trübung (FNU) nach** | | | | |
|---|---|---|---|---|---|
| **Zusatz** | **Start** | **1 Woche** | **2 Woche** | **3 Woche** | **4 Woche** |
| Keiner | 0,39 | 0,70 | 4,46 | 8,74 | 14,11 |
| 0,01 mg/ml Poloxamer | 0,36 | 0,57 | 0,52 | 1,59 | 0,89 |
| 0,015 mg/ml Zn | 1,02 | 0,68 | 0,70 | 0,56 | 0,86 |

Sowohl ein Zusatz von Zink als auch der Zusatz von Poloxamer verhindern das Auftreten von Trübungen im Schütteltest.

### Beispiel 6: Stabilisierung von HMR1964 durch Zusatz von Poloxamer im Rotationstest

a) Zu einer wässrigen Lösung, die in der finalen Formulierung 3,3 mg/ml Phenol, 5 mg/ml NaCl und 6 mg/ml Trometamol enthält wird zinkfreies HMR1964 (so berechnet, dass in der fertigen Formulierung eine Konzentration von 3,5 mg/ml entsteht) gelöst und der pH Wert mit 1 N Salzsäure/1 N NaOH auf 7,2 - 7,4 (gemessen bei Raumtemperatur) eingestellt. Die Lösung wird mit Wasser zum Endvolumen aufgefüllt und über einen 0,2 µm Filter steril filtriert. Anschliessend wird sie in 5 ml Injektionsfläschchen gefüllt und mit Kappen verschlossen.
b) Eine Vergleichslösung wird identisch hergestellt, jedoch wird vor dem Auffüllen mit Wasser eine entsprechende Menge einer 0,1 %igen Poloxamer 171 (Genapol^{®}) Stammlösung hinzugefügt, sodass sich in der fertigen Formulierung eine Konzentration von 100 µg/ml ergibt.

Jeweils 5 Muster werden anschliessend im Rotationstest gestresst und die Trübung nach verschiedenen Zeiträumen bestimmt. Die Ergebnisse sind in nachfolgender Tabelle wiedergegeben.

| | **Anzahl Prüfmuster mit Trübung > 18 FNU nach:** | | | | | |
|---|---|---|---|---|---|---|
| Beschreibung | 0 h | 8 h | 16 h | 24 h | 32 h | 40 h |
| HMR1964 Ohne Zusatz | 0 | 5 | - | - | - | - |
| HMR1964 + 0,10 mg/ml Poloxamer 171 | 0 | 0 | 0 | 0 | 1 | 5 |

Der Zusatz von 100 µg/ml Poloxamer stabilisiert die HMR1964 Zubereitung ebenfalls sehr deutlich.

## Patentansprüche

1. Wässrige pharmazeutische Formulierung enthaltend Asp(B28) Humaninsulin oder Lys (B28) Pro (B29) Humaninsulin;
ein Tensid oder Kombinationen mehrerer Tenside; wobei das Tensid Partial - und Fettsäureester und -ether mehrwertiger Alkohole, des Glycerols und des Sorbitols darstellt; wobei die pharmazeutische Formulierung weniger als 0,2 Gewichtsprozent Zink bezogen auf den Insulingehalt der Zubereitung enthält.

2. Pharmazeutische Formulierung gemäß Anspruch 1, wobei die Partial- und Fettsäureester und -ether der mehrwertigen Alkohole, des Glycerols und Sorbitols ausgewählt werden aus einer Gruppe enthaltend Polysorbate.

3. Pharmazeutische Formulierung gemäß einem der Ansprüche 1 oder 2, wobei das Polysorbat ausgewählt wird aus einer Gruppe enthaltend Polysorbat 20 und Polysorbat 80.

4. Pharmazeutische Formulierung gemäß einem der Ansprüche 1 bis 3, wobei ein Konservierungsmittel enthalten ist, ausgewählt aus einer Gruppe enthaltend Phenol, Kresol, Chlorkresol, Benzylalkohol, Parabene.

5. Pharmazeutische Formulierung gemäß einem der Ansprüche 1 bis 4, wobei ein Isotonisierungsmittel enthalten ist, ausgewählt aus einer Gruppe enthaltend Mannitol, Sorbitol, Lactose, Dextrose, Trehalose, Natriumchlorid, Glycerol.

6. Pharmazeutische Formulierung gemäß einem der Ansprüche 1 bis 5, wobei ein Hilfsstoff enthalten ist ausgewählt aus einer Gruppe enthaltend Puffersubstanzen wie z.B. TRIS, Phosphat, Citrat, Acetat, Glycylglycin oder weiterer Stoffe wie Säuren, Laugen, Salze, Protamin, Arginin.

7. Pharmazeutische Formulierung gemäß einem der vorigen Ansprüche, bei der das Asp(B28) Humaninsulin oder Lys(B28) Pro(B29) Humaninsulin in einer Konzentration von 60 - 6000 nmol/ml vorliegt.

8. Pharmazeutische Formulierung gemäß Anspruch 7, wobei das Asp(B28) Humaninsulin oder Lys(B28) Pro(B29) Humaninsulin in einer Konzentration von 240 - 3000 nmol/ml vorliegt

9. Pharmazeutische Formulierung gemäß einem der vorigen Ansprüche, bei der das Tensid in einer Konzentration von 0,1 - 10000 µg/ml vorliegt.

10. Pharmazeutische Formulierung gemäß Anspruch 9, bei der das Tensid in einer Konzentration von 1 - 1000 µg/ml vorliegt.

11. Pharmazeutische Formulierung gemäß einem oder mehreren der Ansprüche 5 bis 10, bei der Glycerol und/oder Mannitol in einer Konzentration von 100 - 250 mM vorliegt.

12. Pharmazeutische Formulierung gemäß Anspruch 11, bei der Chlorid in einer Konzentration von bis zu 150 mM vorliegt.

13. Pharmazeutische Formulierung gemäß einem der vorigen Ansprüche, bei der eine Puffersubstanz in einer Konzentration von 5 - 250 mM vorliegt.

14. Verfahren zur Herstellung einer pharmazeutische Formulierung gemäß einem oder mehreren der Ansprüche 1 bis 13, wobei die Komponenten in Form wäßriger Lösungen zusammengegeben werden, anschließend der gewünschte pH-Wert eingestellt und auf das Endvolumen mit Wasser aufgefüllt wird.

## Claims

1. An aqueous pharmaceutical formulation comprising Asp(B28) human insulin or Lys(B28) Pro(B29) human insulin;
a surfactant or combinations of a number of surfactants; wherein the surfactant represents partial and fatty acid esters and ethers of polyhydric alcohols, of glycerol and sorbitol; wherein the pharmaceutical formulation comprises less than 0.2 percent by weight of zinc based on the insulin content of the preparation.

2. The pharmaceutical formulation as claimed in claim 1, wherein the partial and fatty acid esters and ethers of polyhydric alcohols, of glycerol and sorbitol are selected from a group comprising polysorbate.

3. The pharmaceutical formulation as claimed in either of claims 1 and 2, wherein the polysorbate is selected from a group comprising polysorbate 20 and polysorbate 80.

4. The pharmaceutical formulation as claimed in any of claims 1 to 3, comprising a preservative selected from a group comprising phenol, cresol, chlorocresol, benzyl alcohol, parabens.

5. The pharmaceutical formulation as claimed in any of claims 1 to 4, comprising an isotonicizing agent selected from a group comprising mannitol, sorbitol, lactose, dextrose, trehalose, sodium chloride, glycerol.

6. The pharmaceutical formulation as claimed in any of claims 1 to 5, comprising an excipient selected from a group comprising buffer substances such as, for example, TRIS, phosphate, citrate, acetate, glycylglycine or further substances such as acids, alkalis, salts, protamine, arginine.

7. The pharmaceutical formulation as claimed in any of the preceding claims, wherein the Asp(B28) human insulin or Lys(B28) Pro(B29) human insulin is present in a concentration of 60 - 6000 nmol/ml.

8. The pharmaceutical formulation as claimed in claim 7, wherein the Asp(B28) human insulin or Lys(B28) Pro(B29) human insulin is present in a concentration of 240 - 3000 nmol/ml.

9. The pharmaceutical formulation as claimed in any of the preceding claims, wherein the surfactant is present in a concentration of 0.1 - 10000 µg/ml.

10. The pharmaceutical formulation as claimed in claim 9, wherein the surfactant is present in a concentration of 1 - 1000 µg/ml.

11. The pharmaceutical formulation as claimed in one or more of claims 5 to 10, wherein glycerol and/or mannitol is present in a concentration of 100 - 250 mM.

12. The pharmaceutical formulation as claimed in claim 11, wherein chloride is present in a concentration of up to 150 mM.

13. The pharmaceutical formulation as claimed in any of the preceding claims, wherein a buffer substance is present in a concentration of 5 - 250 mM.

14. A process for the production of a pharmaceutical formulation as claimed in one or more of claims 1 to 13, wherein the components are mixed together in the form of aqueous solutions, then the desired pH is adjusted and the mixture is made up to the final volume with water.

## Revendications

1. Formulation pharmaceutique aqueuse contenant Asp(B28)-insuline humaine ou Lys(B28),Pro(B29)-insuline humaine ;
un agent tensioactif ou des combinaisons de plusieurs agents tensioactifs ; l'agent tensioactif représentant des esters partiels et des esters d'acides gras et des éthers partiels d'alcools polyvalents, du glycérol et du sorbitol ;
la formulation pharmaceutique contenant moins de 0,2% en poids de zinc par rapport à la teneur en insuline de la composition.

2. Formulation pharmaceutique selon la revendication 1, les esters partiels et les esters d'acides gras et les éthers partiels d'alcools polyvalents, du glycérol et du sorbitol étant choisis dans un groupe contenant les polysorbates.

3. Formulation pharmaceutique selon l'une quelconque des revendications 1 et 2, le polysorbate étant choisi dans un groupe contenant le polysorbate 20 et le polysorbate 80.

4. Formulation pharmaceutique selon l'une quelconque des revendications 1 à 3, contenant un conservateur, choisi dans un groupe contenant le phénol, le crésol, le chlorocrésol, l'alcool benzylique, les parabènes.

5. Formulation pharmaceutique selon l'une quelconque des revendications 1 à 4, contenant un agent d'isotonisation, choisi dans un groupe contenant le mannitol, le sorbitol, le lactose, le dextrose, le tréhalose, le chlorure de sodium, le glycérol.

6. Formulation pharmaceutique selon l'une quelconque des revendications 1 à 5, contenant un adjuvant choisi dans un groupe contenant des substances tampon telles que par exemple le TRIS, le phosphate, le citrate, l'acétate, la glycylglycine ou d'autres substances telles que les acides, les lessives alcalines, les sels, la protamine, l'arginine.

7. Formulation pharmaceutique selon l'une quelconque des revendications précédentes, dans laquelle l'Asp(B28)-insuline humaine ou la Lys(B28),Pro(B29)-insuline humaine se trouve en une concentration de 60-6000 nmoles/ml.

8. Formulation pharmaceutique selon la revendication 7, dans laquelle l'Asp(B28)-insuline humaine ou la Lys(B28),Pro(B29)-insuline humaine se trouve en une concentration de 240-3000 nmoles/ml.

9. Formulation pharmaceutique selon l'une quelconque des revendications précédentes, dans laquelle l'agent tensioactif se trouve en une concentration de 0,1-10 000 µg/ml.

10. Formulation pharmaceutique selon la revendication 9, dans laquelle l'agent tensioactif se trouve en une concentration de 1-1000 µg/ml.

11. Formulation pharmaceutique selon l'une ou plusieurs des revendications 5 à 10, dans laquelle le glycérol et/ou le mannitol se trouve(nt) en une concentration de 100-250 mM.

12. Formulation pharmaceutique selon la revendication 11, dans laquelle le chlorure se trouve en une concentration jusqu'à 150 mM.

13. Formulation pharmaceutique selon l'une quelconque des revendications précédentes, dans laquelle une substance tampon se trouve en une concentration de 5-250 mM.

14. Procédé de préparation d'une formulation pharmaceutique selon l'une ou plusieurs des revendications 1 à 13, dans lequel les composants sont réunis sous forme de solutions aqueuses, puis la valeur de pH souhaitée est réglée et on complète au volume final avec de l'eau.
